# EUROPEAN PATENT SPECIFICATION

(11) **EP 1 305 079 B1**
(45) Date of publication and mention of the grant of the patent: **27.10.2004**
(21) Application number: 00962033.7
(22) Date of filing: 01.08.2000
(51) Int. Cl.: A61M 39/22

(54) **APPARATUS FOR FLUSHING A VASCULAR CATHETER**
VORRICHTUNG ZUM SPÜLEN EINES VASKULÄREN KATHETERS
DISPOSITIF DE RINçAGE POUR CATHETER VASCULAIRE

(43) Date of publication of application: 02.05.2003
(73) Proprietor: MAYO FOUNDATION FOR MEDICAL EDUCATION AND RESEARCH, Rochester, MN 55905 (US)
(72) Inventor: SCHWARTZ, Robert, S., Rochester, MN 55902 (US); HOLMES, David, R., Rochester, MN 55906 (US); BERRY, David, Santa Fe, New Mexico 87508 (US); ELLIS, Donald, G., Colorado Springs, CO 80906 (US)
(74) Representative: Petri, Stellan
(86) International application number: PCT/US2000/040534
(87) International publication number: WO 2002/009804

(56) References cited:
- US-A- 4 200 119
- US-A- 4 341 224
- US-A- 5 334 165
- US-A- 5 740 810
- US-A- 6 117 102

## Description

This invention relates generally to devices used in vascular catheter procedures, and more particularly to devices for flushing a catheter during such procedures.

### Background of the Invention

When performing diagnostic, therapeutic or interventional vascular procedures involving a catheter, it is necessary to flush the catheter (typically with saline) in order to prevent blood clots from forming. The device most commonly used today is a manifold with a number of manually operated stopcock valves. Periodically the physician or technician must manually open or close one or more stopcocks for a period of time so that saline from a high pressure source can flush the catheter. After a satisfactory amount of flushing has been done, the stopcocks are again manually moved back to their original position, and the surgeon then resumes performing the procedure. Documents describing the state of the art are US 5,334,165, US 5,740,810 and US 4,341,224.

There are a number of shortcomings with the existing manifold. One is that the physician is "hands off' from performing the procedure for a long period of time (20 seconds or more per flush), which time could instead be used to perform the procedure on the patient. Having to manually flush also breaks the continuity of the procedure, which can be distracting. It also requires that the operator (if not the physician) is at all times sterile. Another shortcoming is that flushing must be done fairly often. During each flush, blood pressure readings are interrupted, which is undesirable. Each additional flush is also another interruption, potential distraction and further delay of the procedure.

What has been needed is an apparatus for flushing a vascular catheter which automatically closes and which provides a relatively slow continuous flush of the catheter.

### Summary of the Invention

According to the present invention, an apparatus for flushing a vascular catheter is provided. The inventions can be used in a variety of diagnostic, therapeutic and interventional procedures involving a vascular catheter.

In one aspect of the invention, the apparatus comprises a housing having an inlet port for connection to a flushing liquid source, and an outlet port for communication with the vascular catheter. Inlet and outlet passages are in communication with the inlet and outlet ports, respectively, and are also selectively in communication with one another. A manually operable member is biased toward a closed position and moveable to an open position. A sealing member cooperates with the manually operable member such that, in the closed position, the inlet and outlet passages are sealed from one another by the sealing member; in the open position, the inlet and outlet passages are in communication with one another. Thus, when the manually operable member is released, it automatically returns to a closed position. The sealing member is slowed toward a sealed position, thereby flushing the catheter for a period of time after the manually operable member has been moved to the open position.

In another aspect of the invention, the apparatus comprises a housing having an inlet port for connection to a flushing liquid source and an outlet port for communication with the vascular catheter. Mechanisms for continuously and periodically flushing are provided. The continuously flushing mechanism provides a relatively slow rate of flow of flushing fluid from the inlet port to the outlet port. The periodically flushing mechanism bypasses the continuously flushing mechanism to provide a relatively high rate of flow of flushing liquid from the inlet port to the outlet port.

These and other advantages and features of novelty which characterize the invention are pointed out with particularity in the claims annexed hereto. However, for a better understanding of the invention and its advantages, reference should be made to the drawing which forms a further part hereof, and to the accompanying descriptive matter in which there is illustrated and described a preferred embodiment of the invention.

### Brief Description of the Drawing

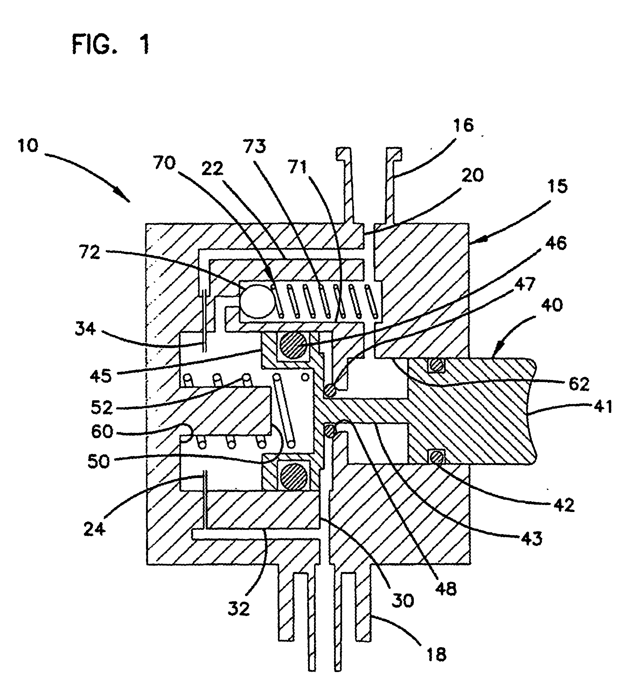

Figure 1 is a cross-sectional view of an apparatus for flushing a vascular catheter according to the present invention.

### Detailed Description of the Preferred Embodiment

Referring now to the drawing, an embodiment of the apparatus of the present invention for flushing a vascular catheter is shown in figure 1.

Apparatus 10 comprises housing 15 including inlet 16 and outlet 18 ports. Inlet port 16 is for connection to a high pressure liquid (typically saline) flushing source as is commonly used. Outlet port 18 is for communication with the vascular catheter, typically through a hemostasis valve.

Apparatus 10 is constructed so that it will automatically return to a closed position after its manually operable member 40 is moved to an open position. Member 40 includes button 41, stem 43, and sealing member 45 configured as a piston, the latter two constituting a plunger. Inlet 20 and outlet 30 passages provide communication between inlet 16 and outlet 18 ports, respectively, and bypass chamber 62. O-rings 42, 47 on button 41 and stem 43 seal chamber 62. When button 41 is depressed, O-ring 47 moves away from seating surface 48, thereby causing flushing liquid to pass from inlet passage 20, through bypass chamber 62 and out outlet port 18 to the catheter. Manually operable member 40 and sealing member 45 are biased toward a closed position (shown) by compression spring 52 on plunger stop 50. Plunger stop 50 defines the end of the open position by sealing member 45 abutting against it.

It will be understood that this arrangement could be varied in a number of ways within the principles of the invention. For example, a lever could be employed instead of a button and plunger. The manually operable member and sealing member also need not be an integral part. A variety of springs (or spring means) could also be employed, and the spring could act on either the operable member or the sealing member.

Apparatus 10 also provides a mechanism by which sealing member 45 is slowed to a closed position so that high pressure flushing fluid continues to flush the catheter after operable member 40 is released. This is accomplished by capillaries 24, 34 and timing chamber 60. Chamber 60 is sealed by O-ring 46 on sealing member 45. When sealing member 45 begins to move out of chamber 60 (to the right in figure), low pressure on the bottom (left in figure) side of sealing member 45 keeps manually operable members 40 from moving back to an extended position. Member 40 gradually moves to the extended position as saline is drawn into timing chamber 60 through capillaries 24, 34, which receive flushing liquid from inlet 20 and outlet 30 passages via capillary inlet 22 and outlet 32 passages. As flushing liquid from capillaries 24, 34 slowly fills timing chamber 60, after a period of time sealing and operable member 40 returns to the extended position, and O-ring 47 closes bypass chamber 62, thereby ending the bypass of high rate liquid flush.

The preferred amount of bypass time is preferably about 20 seconds. The design parameters of the various parts involved could be varied to achieve this. In the preferred version, sealing member 45 has a stroke length of 0.74 cm (0.29 inches) and a diameter of 1.91 cm (0.75 inches). Capillaries 24, 34 are 0.76 cm (0.30 inches) long and have an inside diameter of 0.015 cm (0.0060 inches), resulting in a conductance of approximately 9.5 cm³/(min.bar) (0.04 cubic inches/minute/psi). The mean effective force of spring 52 is about 17.8 N (4 pounds)

It will be understood that a variety of other mechanisms could be employed to slow the movement of the sealing member to the closed position. For example, air from the atmosphere could be drawn through a pin hole into a timing chamber to provide the necessary resistance. Other approaches using air, liquid, another spring means or other mechanisms could also be employed.

Check valve 70 is provided to expel liquid from timing chamber 60 when button 41 is depressed. Check valve 70 includes spring 73 biasing ball 72 to a closed position (shown). When button 41 is depressed, capillaries 24, 34 resist flow through them so that ball 72 unseats and liquid flows from timing chamber 60, through passage 71 and out inlet port 16. A variety of other arrangements could be employed to similarly expel liquid or air from a timing chamber.

Apparatus also provides a relatively slow rate continuous flush of the catheter. This is accomplished by capillaries 24, 34 creating a slow flow from inlet port 16 to outlet port 18 via timing chamber 60. The rate of flow is a slow drip, on the order of 1.0 ml./min. (at 300 mm. Hg pressure drop), resulting in a conductance of 2.37 cm³/(min.bar) (0.010 cubic inches/minute/psi) from the above capillary specifications.

In this way, capillaries 24, 34 serve dual roles. They provide for a slow rate continuous flush, and they also act as the resistance mechanism for slowing the movement of operable member 40 to a bypass closed position.

It will be understood that the apparatus could be modified in a number of ways to provide both continuous flushing and periodic flushing that bypasses the continuous flushing mechanism. A variety of mechanical and electronic means, or a combination thereof, could be employed to achieve this end. Periodic flushing could also be done automatically instead of manually.

It should be understood that the present invention is not limited to the preferred embodiment discussed above, which is illustrative only. Changes may be made in detail, especially in matters of the type, arrangement, shape and size of components within the principles of the invention, to the full extent indicated by the broad general meanings of the terms in which the appended claims are expressed.

## Claims

1. An apparatus for flushing a vascular catheter, comprising:
(a) a housing (15) having an inlet port (16) for connection to a flushing liquid source and an outlet port (18) for communication with the vascular catheter;
(b) inlet (20) and outlet (30) passages in communication with said inlet and outlet ports, respectively, and selectably in communication with one another;
(c) a manually operable member (40) biased toward a closed position and movable to an open position; and
(d) a sealing member (45) cooperating with said manually operable member, wherein in said closed position said inlet and outlet passages are sealed from one another by said sealing member, and in said open position said inlet and outlet passages are in communication with one another;
**characterized by** said sealing member constructed and arranged such that a movement of said sealing member toward a sealed position is substantially slowed, thereby flushing the vascular catheter for a period of time after said manually operable member has been moved to said open position.

2. An apparatus for flushing a vascular catheter according to claim 1, wherein said manually operable member is a button movable between extended and depressed positions corresponding to said closed and open positions respectively.

3. An apparatus for flushing a vascular catheter according to claim 2, wherein said sealing member is a plunger having a sealing gasket around it, said plunger being rigidly connected to said button.

4. An apparatus for flushing a vascular catheter according to claim 1, wherein said manually operable member is biased toward said closed position by a spring.

5. An apparatus for flushing a vascular catheter according to claim 1, wherein said movement of said sealing member toward said sealed position is slowed by means for creating a temporary differential pressure pulling said sealing member away from said sealed position.

6. An apparatus for flushing a vascular catheter according to claim 5, wherein said pressure differential creating means comprise a sealed chamber having an inlet into which fluid slowly flows.

7. An apparatus for flushing a vascular catheter according to claim 6, wherein said apparatus is constructed and arranged such that said fluid is flushing liquid received from said inlet port.

8. An apparatus for flushing a vascular catheter according to claim 6, further including means for expelling fluid from said chamber when said sealing member is moved into said chamber.

9. An apparatus for flushing a vascular catheter according to claim 1, further including means for continuously flushing the vascular catheter by providing a relatively slow rate of flow of flushing liquid from said inlet port to said outlet port.

10. An apparatus for flushing a vascular catheter according to claim 9, wherein said continuously flushing means are also employed to slow a movement of said sealing member to a sealed position.

11. An apparatus for flushing a vascular catheter, comprising:
(a) a housing (15) having an inlet port (16) for connection to a flushing liquid source and an outlet port (18) for communication with the vascular catheter;
(b) means for continuously flushing the vascular catheter by providing a relatively slow rate of flow of flushing liquid from said inlet port to said outlet port;
(c) means for periodically flushing the vascular catheter by bypassing said continuously flushing means to provide a relatively high rate of flow of flushing liquid from said inlet port to said outlet port;
**characterized by** said periodic flushing means including a manually operable member (40) constructed and arranged such that, after being moved to a bypass position, a movement of said member toward a closed position is substantially slowed, thereby continuing to flush the vascular catheter at a relatively high rate for a period of time after said manually operable member has been moved to said bypass position.

12. An apparatus for flushing a vascular catheter according to claim 11, wherein said continuous flushing means comprise a capillary tube providing flushing fluid at a rate of about between 0.10 and 10.0 cubic centimeters per minute.

13. An apparatus for flushing a vascular catheter according to claim 11, wherein said manually operable member is a button biased toward said closed position by a compression spring.

14. An apparatus for flushing a vascular catheter according to claim 11, wherein said movement of said manually operable member toward said closed position is slowed by means for creating a temporary pressure differential pulling said manually operable member away from said closed position.

15. An apparatus for flushing a vascular catheter according to claim 14, wherein said pressure differential creating means comprise a sealed chamber having an inlet into which fluid slowly flows.

16. An apparatus for flushing a vascular catheter according to claim 15, wherein said pressure differential creating means comprise at least a portion of said continuously flushing means.

17. An apparatus for flushing a vascular catheter according to claim 11, wherein said apparatus is constructed and arranged such that said period of time is at least 5 seconds.

18. An apparatus for flushing a vascular catheter according to claim 1, wherein said apparatus is constructed and arranged such that said period of time is at least 5 seconds.

## Patentansprüche

1. Vorrichtung zum Spülen eines vaskulären Katheters, welche umfaßt:
(a) ein Gehäuse (15) mit einer Einlaßöffnung (16) zur Verbindung mit einer Spülflüssigkeitsquelle und einer Auslaßöffnung (18) zur Verbindung mit dem vaskulären Katheter;
(b) Einlaß(20)- und Auslaß(30)durchgänge in Verbindung mit besagten Einlaß- bzw. Auslaßöffnungen und wählbar in Verbindung miteinander;
(c) ein von Hand betätigbares Teil (40), das auf eine geschlossene Position hin vorgespannt und in eine offene Position bewegbar ist; und
(d) ein Dichtungsteil (45), das mit besagtem von Hand betätigbaren Teil zusammenwirkt, wobei besagte Einlaß- und Auslaßdurchgänge in besagter geschlossenen Position durch besagtes Dichtungsteil voneinander abgedichtet sind und besagte Einlaß- und Auslaßdurchgänge in besagter offenen Position in Verbindung miteinander stehen;
**dadurch gekennzeichnet, daß** besagtes Dichtungsteil so konstruiert und angeordnet ist, daß eine Bewegung besagten Dichtungsteils auf eine abgedichtete Position hin beträchtlich verlangsamt wird, wodurch der vaskuläre Katheter für einen Zeitraum gespült wird, nachdem besagtes von Hand betätigbares Teil in besagte offene Position bewegt worden ist.

2. Vorrichtung zum Spülen eines vaskulären Katheters nach Anspruch 1, **dadurch gekennzeichnet, daß** besagtes von Hand betätigbares Teil ein Knopf ist, der zwischen ausgefahrenen und heruntergedrückten Positionen bewegbar ist, die besagten geschlossenen bzw. offenen Positionen entsprechen.

3. Vorrichtung zum Spülen eines vaskulären Katheters nach Anspruch 2, **dadurch gekennzeichnet, daß** besagtes Dichtungsteil ein Stempel mit einem Dichtring um diesen herum ist, wobei besagter Stempel starr mit besagtem Knopf verbunden ist.

4. Vorrichtung zum Spülen eines vaskulären Katheters nach Anspruch 1, **dadurch gekennzeichnet, daß** besagtes von Hand betätigbares Teil durch eine Feder auf besagte geschlossene Position hin vorgespannt ist.

5. Vorrichtung zum Spülen eines vaskulären Katheters nach Anspruch 1, **dadurch gekennzeichnet, daß** besagte Bewegung besagten Dichtungsteils auf besagte abgedichtete Position hin mit Mitteln zur Erzeugung eines temporären Differenzdrucks verlangsamt wird, der besagtes Dichtungsteil von besagter abgedichteten Position wegzieht.

6. Vorrichtung zum Spülen eines vaskulären Katheters nach Anspruch 5, **dadurch gekennzeichnet, daß** besagte Mittel zur Erzeugung eines Druckunterschiedes eine abgedichtete Kammer mit einem Einlaß umfassen, in die langsam Fluid einströmt.

7. Vorrichtung zum Spülen eines vaskulären Katheters nach Anspruch 6, **dadurch gekennzeichnet, daß** besagte Vorrichtung so konstruiert und angeordnet ist, daß besagtes Fluid Spülflüssigkeit ist, die von besagter Einlaßöffnung erhalten wird.

8. Vorrichtung zum Spülen eines vaskulären Katheters nach Anspruch 6, die weiter Mittel zum Ausstoßen von Fluid aus besagter Kammer einschließt, wenn besagtes Dichtungsteil in besagte Kammer hinein bewegt wird.

9. Vorrichtung zum Spülen eines vaskulären Katheters nach Anspruch 1, die weiter Mittel zum kontinuierlichen Spülen des vaskulären Katheters einschließt, durch Bereitstellen einer relativ langsamen Durchflußgeschwindigkeit von Spülflüssigkeit von besagter Einlaßöffnung zu besagter Auslaßöffnung.

10. Vorrichtung zum Spülen eines vaskulären Katheters nach Anspruch 9, **dadurch gekennzeichnet, daß** besagte Mittel zum kontinuierlichen Spülen ebenfalls eingesetzt werden, um eine Bewegung besagten Dichtungsteils in eine abgedichtete Position zu verlangsamen.

11. Vorrichtung zum Spülen eines vaskulären Katheters, welche umfaßt:
(a) ein Gehäuse (15) mit einer Einlaßöffnung (16) zur Verbindung mit einer Spülflüssigkeitsquelle und einer Auslaßöffnung (18) zur Verbindung mit dem vaskulären Katheter;
(b) Mittel zum kontinuierlichen Spülen des vaskulären Katheters durch Bereitstellen einer relativ langsamen Durchflußgeschwindigkeit von Spülflüssigkeit von besagter Einlaßöffnung zu besagter Auslaßöffnung;
(c) Mittel zum periodischen Spülen des vaskulären Katheters durch Umgehen besagter Mittel zum kontinuierlichen Spülen, um eine relativ hohe Durchflußgeschwindigkeit von Spülflüssigkeit von besagter Einlaßöffnung zu besagter Auslaßöffnung bereitzustellen;
**dadurch gekennzeichnet, daß** besagte Mittel zum periodischen Spülen ein von Hand betätigbares Teil (40) einschließen, das so konstruiert und angeordnet ist, daß, nachdem es in eine Umlenkposition bewegt worden ist, eine Bewegung besagten Teils auf eine geschlossene Position hin beträchtlich verlangsamt wird, wodurch das Spülen des vaskulären Katheters bei einer relativ hohen Geschwindigkeit für einen Zeitraum fortgesetzt wird, nachdem besagtes von Hand betätigbares Teil in besagte Umlenkposition bewegt worden ist.

12. Vorrichtung zum Spülen eines vaskulären Katheters nach Anspruch 11, **dadurch gekennzeichnet, daß** besagte Mittel zum kontinuierlichen Spülen ein Kapillarrohr umfassen, das Spülflüssigkeit in einer Geschwindigkeit von etwa 0,10 bis 10,0 Kubikzentimetern pro Minute bereitstellt.

13. Vorrichtung zum Spülen eines vaskulären Katheters nach Anspruch 11, **dadurch gekennzeichnet, daß** besagtes von Hand betätigbares Teil ein Knopf ist, der durch eine Druckfeder auf besagte geschlossene Position hin vorgespannt ist.

14. Vorrichtung zum Spülen eines vaskulären Katheters nach Anspruch 11, **dadurch gekennzeichnet, daß** besagte Bewegung besagten von Hand betätigbaren Teils auf besagte geschlossene Position hin durch Mittel zur Erzeugung eines temporären Druckunterschiedes verlangsamt wird, der besagtes von Hand betätigbares Teil aus besagter geschlossenen Position wegzieht.

15. Vorrichtung zum Spülen eines vaskulären Katheters nach Anspruch 14, **dadurch gekennzeichnet, daß** besagte Mittel zur Erzeugung eines Druckunterschiedes eine abgedichtete Kammer mit einem Einlaß umfassen, in die langsam Fluid einströmt.

16. Vorrichtung zum Spülen eines vaskulären Katheters nach Anspruch 14, **dadurch gekennzeichnet, daß** besagte Mittel zur Erzeugung eines Druckunterschiedes wenigstens einen Abschnitt besagter Mittel zum kontinuierlichen Spülen umfassen.

17. Vorrichtung zum Spülen eines vaskulären Katheters nach Anspruch 11, **dadurch gekennzeichnet, daß** besagte Vorrichtung so konstruiert und angeordnet ist, daß besagter Zeitraum wenigstens 5 Sekunden beträgt.

18. Vorrichtung zum Spülen eines vaskulären Katheters nach Anspruch 1, **dadurch gekennzeichnet, daß** besagte Vorrichtung so konstruiert und angeordnet ist, daß besagter Zeitraum wenigstens 5 Sekunden beträgt.

## Revendications

1. Appareil pour purger un cathéter vasculaire, comprenant :
(a) un boîtier (15) ayant un port d'entrée (16) pour connexion à une source de liquide de purge et un port de sortie (18) pour communication avec le cathéter vasculaire ;
(b) des passages d'entrée (20) et de sortie (30) en communication avec lesdits ports d'entrée et de sortie, respectivement, et en communication de manière sélectionnable l'un avec l'autre ;
(c) un membre (40) utilisable manuellement biaisé vers une position fermée et pouvant être déplacé en une position ouverte ; et
(d) un membre de scellement (45) coopérant avec ledit membre utilisable manuellement, dans lequel dans ladite position fermée lesdits passages d'entrée et de sortie sont séparés l'un de l'autre par ledit membre de scellement, et dans ladite position ouverte lesdits passages d'entrée et de sortie sont en communication l'un avec l'autre ; **caractérisé par** ledit membre de scellement étant construit et arrangé de telle sorte qu'un mouvement du dit membre de scellement vers une position scellée est sensiblement ralenti, purgeant ainsi le cathéter vasculaire pendant une certaine durée après que ledit membre utilisable manuellement a été déplacé dans ladite position ouverte.

2. Appareil pour purger un cathéter vasculaire selon la revendication 1, dans lequel ledit membre utilisable manuellement est un bouton pouvant être déplacé entre des positions étendue et enfoncée correspondant aux dites positions fermée et ouverte respectivement.

3. Appareil pour purger un cathéter vasculaire selon la revendication 2, dans lequel ledit membre de scellement est un piston ayant un joint étanche l'entourant, ledit piston étant connecté de manière rigide au dit bouton.

4. Appareil pour purger un cathéter vasculaire selon la revendication 1, dans lequel ledit membre utilisable manuellement est biaisé vers ladite position fermée par un ressort.

5. Appareil pour purger un cathéter vasculaire selon la revendication 1, dans lequel ledit mouvement du dit membre de scellement vers ladite position scellée est ralenti par un moyen pour créer une pression différentielle temporaire tirant ledit membre de scellement en l'écartant de ladite position de scellement.

6. Appareil pour purger un cathéter vasculaire selon la revendication 5, dans lequel ledit moyen de création de pression différentielle comprend une chambre scellée ayant une entrée dans laquelle un fluide s'écoule lentement.

7. Appareil pour purger un cathéter vasculaire selon la revendication 6, dans lequel ledit appareil est construit et arrangé de telle sorte que ledit fluide est un liquide de purge provenant du dit port d'entrée.

8. Appareil pour purger un cathéter vasculaire selon la revendication 6, comprenant en outre un moyen pour expulser le liquide de ladite chambre lorsque ledit membre de scellement entre dans ladite chambre.

9. Appareil pour purger un cathéter vasculaire selon la revendication 1, comprenant en outre un moyen pour purger de manière continue le cathéter vasculaire en fournissant un débit relativement lent de liquide de purge depuis ledit port d'entrée vers ledit port de sortie.

10. Appareil pour purger un cathéter vasculaire selon la revendication 9, dans lequel ledit moyen de purge continue est aussi utilisé pour ralentir un mouvement du dit membre de scellement vers une position scellée.

11. Appareil pour purger un cathéter vasculaire comprenant :
(a) un boîtier (15) ayant un port d'entrée (16) pour connexion à une source de liquide de purge et un port de sortie (18) pour communication avec le cathéter vasculaire ;
(b) un moyen pour purger de manière continue le cathéter vasculaire en fournissant un débit relativement lent de liquide de purge depuis ledit port d'entrée vers ledit port de sortie ;
(c) un moyen pour purger de manière périodique le cathéter vasculaire en évitant ledit moyen de purge continue pour fournir un débit relativement élevé de liquide de purge depuis ledit port d'entrée vers ledit port de sortie, **caractérisé par** le moyen de purge périodique incluant un membre utilisable manuellement (40) construit et arrangé de telle sorte que, après avoir été déplacé en position d'évitement, un mouvement du dit membre vers une position fermée soit sensiblement ralenti, continuant ainsi à purger le cathéter vasculaire à une vitesse relativement élevée pendant une certaine durée après que ledit membre utilisable manuellement a été déplacé en ladite position d'évitement.

12. Appareil pour purger un cathéter vasculaire selon la revendication 11, dans lequel ledit moyen de purge continue comprend un tube capillaire fournissant du fluide de purge à une vitesse d'environ 0,10 à 10,0 centimètres cubes par minute.

13. Appareil pour purger un cathéter vasculaire selon la revendication 11, dans lequel ledit membre utilisable manuellement est un bouton biaisé vers ladite position fermée par un anneau de compression.

14. Appareil pour purger un cathéter vasculaire selon la revendication 11, dans lequel ledit mouvement du dit membre utilisable manuellement vers ladite position fermée est ralenti par un moyen pour créer un différentiel de pression temporaire tirant ledit membre utilisable manuellement en l'écartant de ladite position fermée.

15. Appareil pour purger un cathéter vasculaire selon la revendication 14, dans lequel ledit moyen de création d'un différentiel de pression comprend une chambre scellée ayant une entrée par laquelle le fluide s'écoule lentement.

16. Appareil pour purger un cathéter vasculaire selon la revendication 15, dans lequel ledit moyen de création d'un différentiel de pression comprend au moins une partie du dit moyen de purge continue.

17. Appareil pour purger un cathéter vasculaire selon la revendication 11, dans lequel ledit appareil est construit et arrangé de telle sorte que ladite durée est d'au moins cinq secondes.

18. Appareil pour purger un cathéter vasculaire selon la revendication 1, dans lequel ledit appareil est construit et arrangé de telle sorte que la dite durée est d'au moins cinq secondes.
